# EUROPEAN PATENT SPECIFICATION

(11) **EP 0 392 548 B1**
(45) Date of publication and mention of the grant of the patent: **19.10.1994**
(21) Application number: 90107068.0
(22) Date of filing: 12.04.1990
(51) Int. Cl.: A61B 17/28

(54) **Multi-position latching mechanism for forceps**
Schnappverschlussvorrichtung mit mehreren Stellungen für Zange
Mécanisme de fermeture à loquet à plusieurs positions pour pince

(30) Priority: 14.04.1989 US 339042
(43) Date of publication of application: 17.10.1990
(73) Proprietor: CODMAN & SHURTLEFF INC., Randolph Massachusetts 02368 (US)
(72) Inventor: Santangelo, John A., East Freetown, MA 02717 (US)
(74) Representative: Strehl Schübel-Hopf Groening & Partner

(56) References cited:
- DE-A- 3 103 352
- DE-C- 842 112
- GB-A- 2 117 693
- US-A- 2 652 832
- US-A- 4 397 312
- US-A- 4 800 880
- US-A- 4 823 792

## Description

### FIELD OF THE INVENTION

The present invention relates to a latching mechanism for a forceps-type surgical instrument and, more particularly, to a multi-position latching mechanism for an aneurysm clip applying forceps. The latching mechanism holds the forceps jaws at various predetermined spacings to facilitate the loading of clips into the jaws and the placement of clips on the anatomy about the surgical site.

### BACKGROUND OF THE INVENTION

Forceps are used to place a variety of clips on the anatomy during surgery. A variety of clips usually hemostatic clips are applied with forceps. One type of hemostatic clip of particular interest is an aneurysm clip. One type of aneurysm clip is shown in U.S. Patent No. 4,777,950.

Hemostatic clips, including aneurysm clips, are applied to blood vessels in various ways to close or strengthen a blood vessel during or after surgery. The jaws of a forceps are usually designed to receive a particular type of clip. A clip is loaded into the jaws by the surgical assistant and then handed to the surgeon who inserts the clip into the surgical site.

To load a clip into the jaws the surgical assistant opens the jaws and places the clip between the jaws manually and then closes the jaws enough to hold the clip in place so that it will not fall out during insertion. It is, however, important that the jaws not be closed too much so as to start to open the clip since that might interfere with the proper insertion of the clip.

One way of setting the jaws at a predetermined position is to use two projections from the inside of each handle of the forceps which overlap and interlock as is shown for example in U.S. Patent No. 3,393,680. Although this is a satisfactory lock for some applications, it is not particularly well suited to applying an aneurysm clip. A leaf spring extending between the insides of the two opposing handles of the forceps is used to bias the handles apart so that when the lock is disengaged the forceps handles will open a predetermined amount thus opening the jaws of the corresponding predetermined amount.

Another kind of forceps locking mechanism is shown in U.S. Patent No. 4,462,404. This forceps locking mechanism can be used to place the jaws at multiple positions. A leaf spring attached to the inside of one forceps handle extends across to the inside of the other forceps handle and engages a complex latch which is affixed to the inside of the opposing handle. Although this device works satisfactorily, it is complicated and has variety of parts which must be separately assembled to the forceps. Care must be taken with this kind of latch mechanism to make sure that it does not become damaged and misaligned during cleaning and sterilization of the forceps.

It would be useful to have a latching device of simpler design which could be used to set the jaws of the forceps in a variety predetermined spaced apart positions so that the surgical assistant could first load the clip into the jaws and then firmly set the clip into position without activating the clip. This would allow the surgical assistant to quickly load the clip and hand the instrument surgeon who could easily insert the clip into the surgical site. It would also be useful to have the latch recycled to its initial position as the surgeon releases his grasp on the forceps to further facilitate the loading of the next clip by the surgical assistant or repositioning or removal of a clip just placed without withdrawing the applier from the surgical site. Quick, one hand recycling of the latch mechanism would be very useful in repositioning or removing a clip.

### SUMMARY OF THE INVENTION

The present invention relates to multi-position latching mechanism for a forceps type surgical instrument.

The latching mechanism has at least a first position to place the jaws in a first predetermined spacing and a second position to place the jaws in a second predetermined spacing. The mechanism includes a retaining means on one of the handles which is preferably a V-notch where according to an embodiment of the invention the proximal portion of the V-notch forms an acute angle with the inside surface of the handle into which it is placed and the distal portion of the V-notch forms an obtuse angle with the inside surface of the handle into which it is placed. The retaining means may be one or more V notches or other retaining structures recessed into or raised above the inside surface of the handle for retaining the latch mechanism.

The latching mechanism has a latch attached to one of the forceps handles and is resiliently flexible in the plane of motion of the handles. The latch is preferably made of spring steel with a proximal portion attached to one handle and the distal portion extending at least partially about the retaining means.

The latching mechanism has a hook attached to the other handle which is resiliently flexible in the plane of motion of the handles. The hook has a proximal end attached to its handle and a distal end.

The latching mechanism has a biasing mechanism for placing the handles in a first predetermined position. The hook and the retaining means cooperate to place the handles in a second predetermined position upon further closing of the handles. The hook and the latch cooperate to recycle upon further closing and then opening of the handles to return the jaws to the first predetermined position without engaging the retaining means again.

Preferably, both the latch and the hook have an L-shaped projection extending from their distal end. This projection from the distal end of the latch or the hook need not be L-shaped. All that is important is that there be a cross bar supported from the distal end of the latch and the hook and that there be a connection between the cross bar and the distal end of the latch or the hook.

According to an embodiment of the invention the surface of the latch cross bar facing the space confined by the insides of the handles forms a convex curve as one proceeds from the distal to the proximal portion of the cross bar. Preferably, the distal end of the hook includes a projection extending toward the inside of the handle containing the retaining means, which projection forms an acute angle with the remainder of the cross bar of the hook. This angled projection is intended to engage the retaining means if the retaining means is formed in the shape of the V-shaped notch.

The instrument may include a pair of leaf springs extending rearwarding from the proximal end of each handle which include a slot and a tab at their respective proximal ends for connecting the two leaf springs together. Preferably, one leaf spring is integrally formed with the hook and the other leaf spring is integrally formed with the latch. Alternatively, these leaf springs may be placed between the handles or may be replaced by a compression spring or a torsion spring located between the handles, preferably near the pivot point of the handles.

As the latching mechanism moves among its various positions, audible clicks and/or tactile signals may inform the user of the operation of the latching mechanism.

These and other features and advantages of the present invention will become more apparent from the following detailed description of the preferred embodiment taken in conjunction with the following drawings.

### BRIEF DESCRIPTION OF THE DRAWINGS

Figure 1 shows a side elevation of the forceps and latching mechanism of the present invention with an aneurysm clip placed in the jaws with phantom lines indicating that as the jaws close the clip opens;
Figure 2 shows a partial side elevation of the latching mechanism in one position;
Figure 3 shows a partial perspective of the latching mechanism in the position of Figure 2;
Figure 4 shows a partial perspective of a portion of the forceps of Figure 1;
Figure 5 shows a partial side elevation of the latching mechanism as the jaws close from the position of Figure 3;
Figure 6 shows a partial perspective of the latching mechanism in the position of Figure 5;
Figure 7 shows a partial side elevation of the latching mechanism in a still different position;
Figure 8 shows a partial perspective of the latching mechanism in the position of Figure 7;
Figure 9 shows a partial perspective view of an alternate embodiment of the latch using a different latch and showing multiple notches;
Figure 10 shows a partial perspective view of still another alternate embodiment of the latch;
Figure 11 shows a partial perspective view of another alternate embodiment of the invention using a straight latch with the notches recessed below the surface of the handle; and,
Figure 12 shows a partial side view of the embodiment of Figure 11.

### DETAILED DESCRIPTION OF THE PREFERRED EMBODIMENTS

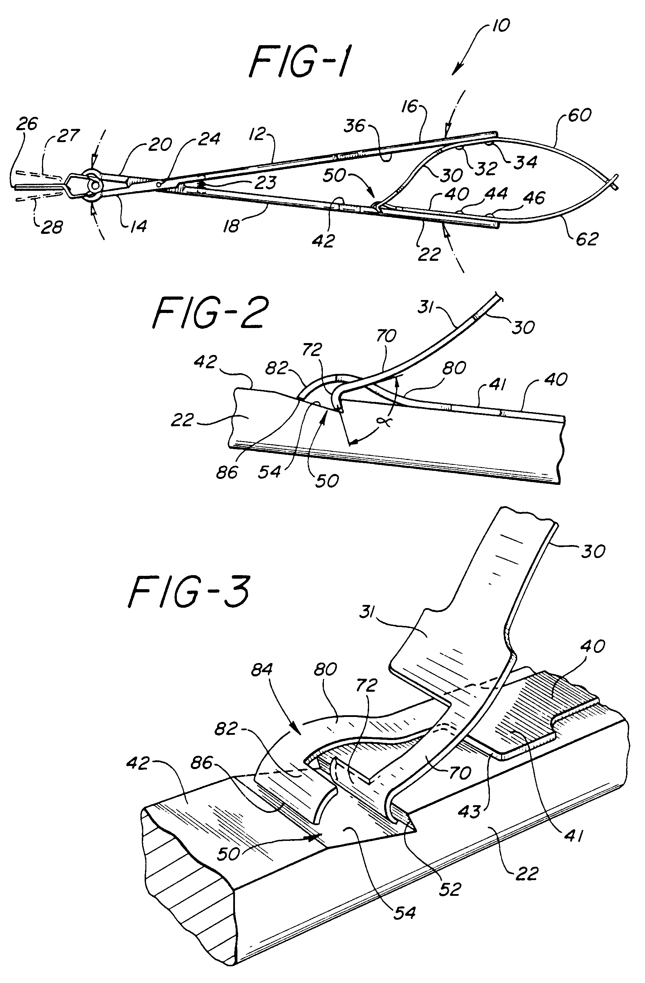

Referring now to Figure 1 there is shown a forceps 10 having a first member 12 with a jaw 14 on its distal end and a handle 16 on its proximal end and a second member 18 with a jaw 20 on its distal end and a handle 22 on its proximal end. Members 12 and 18 are pivotably connected together at pivot point 24.

Jaws 14 and 20 are specially configured to hold an aneurysm clip 26. A hook member 30 is attached by suitable means, preferably screws 32 and 34, to the inside 36 of the distal portion of handle 16. Hook member 30 is preferably a piece of flat spring steel but may be made in any convenient shape or of any suitable resilient material so that hook member 30 may be resilient in the plane of motion of handles 16 and 22.

It will be noted that handles 16 and 22 may be offset from the body of members 12 and 18 to form what is known in the surgical instrument vernacular as a bayonnet style forceps such that the plane in which jaws 14 and 20 move is laterally offset from the plane in which handles 16 and 22 move. This offset bayonnet design is convenient for such instruments, but not essential.

In the preferred embodiment, a latch 40 is affixed along the inside 42 of handle 22 at a proximal portion of handle 22 by means of screws 44 and 46.

The latch mechanism also includes a retaining means 50 which is cut into inside surface 42 of handle 22. As seen best in Figures 2 and 3, retaining means 50 is preferably a V-shaped notch whose proximal portion 52 forms an acute angle with the adjacent inside wall 42 of handle 22 and whose distal portion 54 forms an obtuse angle with the adjacent portion of inside wall 42 of handle 22. In alternative embodiments, notch 50 need not be cut into surface 42 of handle 22 but could be a raised projection extending above inside surface 42 or some other suitable mechanism.

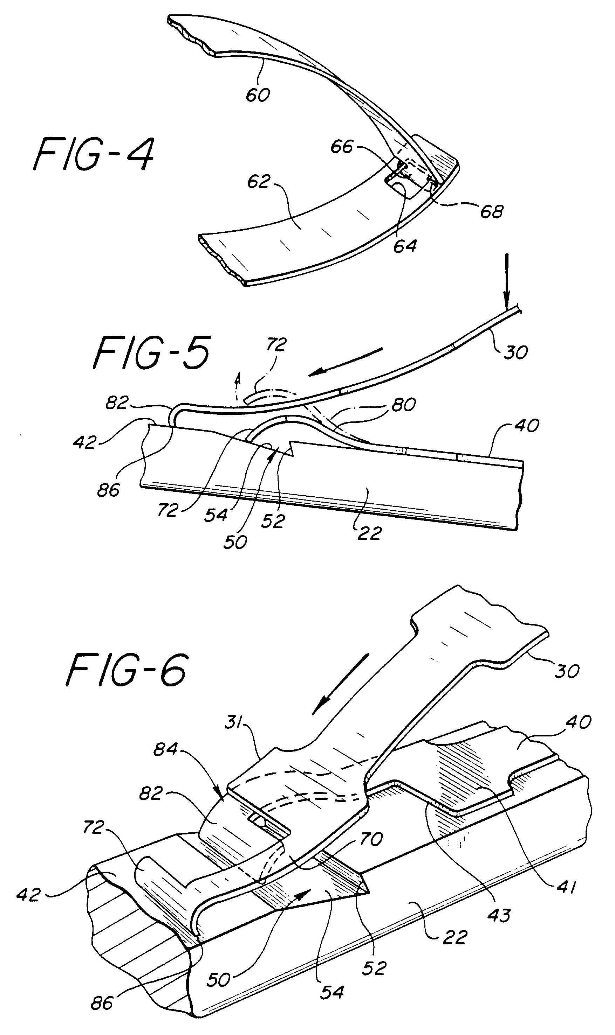

Referring again to Figure 1, one will notice that hook 30 has a proximal extension 60 and latch 40 has a proximal extension 62 which are both preferably made integrally of the same spring steel as their respective hook 30 and latch 40. Figure 4 shows that extension 62 includes a slot 64 in its most proximal portion and extension 60 includes a tab 66 at its most proximal portion which has an expanded head 68. Slot 64 and tab 66 connect together to hold extensions 60 and 62 together to provide a spring force to bias handles 16 and 22 apart a predetermined amount.

Referring now to Figure 3, we will now describe the details of the latching mechanism. Hook 30 includes a distally extending L-shaped projection with a shaft 70 and cross bar 72. Cross bar 72 extends transversely from the distal end of shaft 70. Cross bar 72 is preferably arranged at an angle (α) (see Figure 2) to shaft 70 to provide a hook to hold hook 30 in notch 50. Angle (α) is preferably close to 90° and preferably mated to the acute angle which the proximal portion 52 of V-notch 50 forms with the inside wall 42 of handle 22. Any convenient combination of angles can be used so that hook 30 is held satisfactorily in notch 50.

Projection 70 from the distal portion of hook 30 need not be L-shaped but may be any convenient shape so long as cross bar 72 is held in the position that will permit it to easily enter V-notch 50 when necessary. For example, shaft 70 could be C-shaped instead of a straight shaft or a projection from the side of hook 30 or any other shape that provided a convenient connection between cross bar 72 and distal portion 31 of hook 30.

Still referring to Figure 3, one can see that latch 40 has a similar L-shaped projection extending from the distal portion 41 of latch 40 with a shaft 80 and a cross bar 82. Shaft 80 extends along one side of surface 42 of handle 22 and shaft 70 extends along the other side. Cross bar 82 extends across surface 42 of handle 22 in one direction, whereas cross bar 72 extends across surface 42 of handle 22 in the opposite direction so that as cross bar 72 moves distally upon the closing of handle 16 toward handle 22, cross bar 72 will recycle under cross bar 82. Distal surface 43 of distal portion 41 can serve as a retainer to hold cross bar 72 to place jaws 14, 20 in a desired spacing.

Still referring to Figure 3, one will note that the upper surface 84 of cross bar 82 may include a convex curve to assist in recycling cross bar 72. As with shaft 70 of hook 30, shaft 80 of latch 40 need not be straight, but may be any convenient shape to provide a connection between the distal portion 41 of latch 40 and cross bar 82.

It will be noted that cross bar 82 extends beyond proximal surface 52 of notch 50 so that the L-shaped projection of latch 40 extends at least partly about notch 50.

It is important, as shown in Figure 3, that the free end of cross bar 72 of hook 30 extends far enough across surface 42 to contact cross bar 82 during use, but not so far as to contact shaft portion 80. The particular shape of cross bars 72 and 82 is unimportant. All that is important is that cross bars 72 and 82 can interact with one another with 72 traveling out notch 50 under 82 as handles 16 and 22 move toward one another and then back over 82 without reengaging notch 50 as handles 16 and 22 release. The distal edge 86 (see Fig. 2) of cross bar 82 may be biased in resilient contact with surface 42 of handle 22 and preferably biased in resilient contact with the proximal surface of V-shaped notch 50.

The operation of the latching mechanism will now be explained in connection with Figures 3, 6 and 8.

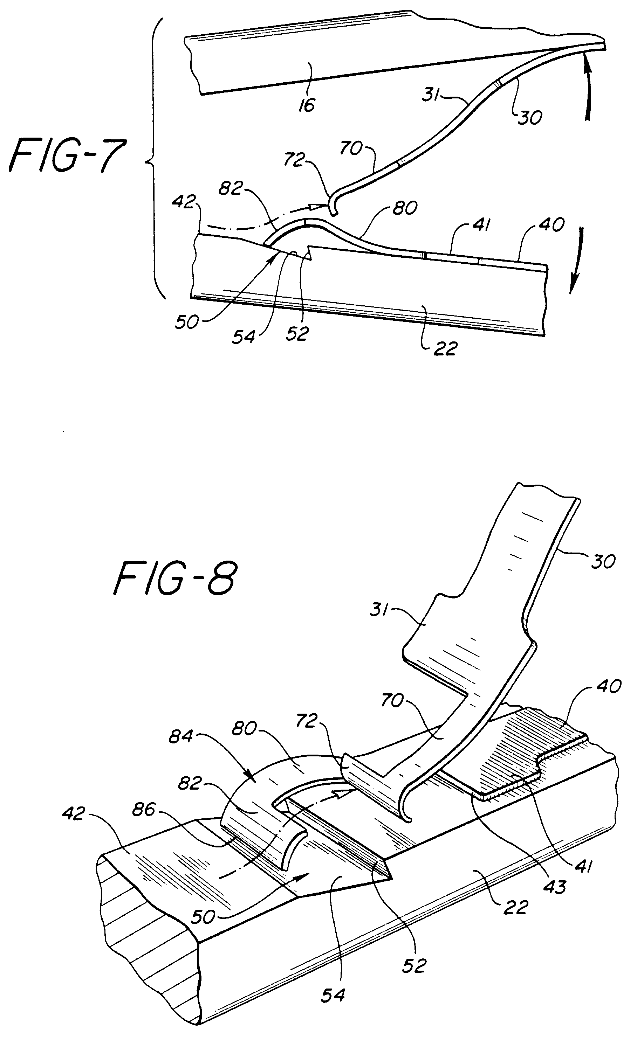

Referring first to Figure 8, one will notice that cross bar 72 of hook 30 is disposed proximally of notch 50 and preferably rests against surface 42, however, it is not necessary to have cross bar 72 rest against surface 42. With the latching mechanism in the position shown in Figure 8, the spacing of jaws 14 and 20 is controlled by extensions 60 and 62. If cross bar 72 were resting against surface 42 of handle 22 the spacing of jaws 14 and 20 could be controlled by the resiliency of hook 30 or by a combination of the resiliency of hook 30 and extensions 60 and 62. Those skilled in the art will recognize that this initial spacing of the jaws can be properly controlled by adjusting hook 30 and extensions 60 and 62. This is important because these parts can become bent and misaligned during cleaning and sterilization, but it is easy to realign them to allow the latching mechanism to work properly. In this initial position the surgical assistant may insert an aneurysm clip 26 between jaws 14 and 20.

Once aneurysm clip 26 is in position between the jaws, the surgical assistant partially closes the handles causing cross bar 72 of hook 30 to move distally and down toward surface 42 until it rides into notch 50. The principal force which causes cross bar 72 to hook onto notch 50 is the resiliency of hook 30.

As cross bar 72 drops into notch 50 an audible click and a tactile signal are made so that the surgical assistant knows that aneurysm clip 26 is firmly held in position between jaws 14 and 20, but the jaws of the aneurysm clip 27 and 28 shown in phantom in Figure 1 are not opened.

The surgical assistant can then pass the forceps 10 to the surgeon who grasps handles 16 and 22 and inserts the jaws and the aneurysm clip into the surgical site. To open jaws 27 and 28 of aneurysm clip 26 the surgeon further closes handles 16 and 22 together.

Referring now to Figure 6, it can be seen that the further closing of handles 16 and 22 together causes cross bar 72 to travel distally under cross bar 82 deflecting cross bar 82 away from surface 42 (see particularly Figure 5 where the upwardly deflected position of cross bar 82 is shown in phantom). As soon as cross bar 72 clears cross bar 82, cross bar 82 resiliently deflects back into contact with surface 42 of handle 22 making an audible or tactile signal indicating that the latch mechanism is free and that jaws 14 and 20 are closed and jaws 27 and 28 of clip 26 are now partly open. Jaws 14 and 20 close further allowing jaws 27 and 28 of aneurysm clip 26 to open so that they may be placed about the anatomy of interest. The surgeon may then close jaws 27 and 28 together about the anatomy of interest by relaxing his grip on handles 16 and 22 so that jaws 14 and 16 open to release clip 26. As jaws 14 and 20 open and handles 16 and 22 move apart, cross bar 72 moves distally along surface 42 and rides over convex surface 84 of cross bar 82 (see Figures 7 and 8). As the handles 16 and 22 open further cross bar 72 of hook 30 move proximally of notch 50 passing over notch 50 without engaging proximal surface 52 of notch 50. Further audible and tactile and visible signals (hook 30 rests behind notch 50) indicate to the surgeon that the jaws are now open so that the forceps 10 may be withdrawn from the incision without tugging on clip 26 and stressing the anatomy to which it is attached.

One will note from Figures 7 and 8 that cross bar 72 of hook 30 is angled at a sufficient angle α so that even if handles 16 and 22 are opened very slowly and cross bar 72 passes very slowly over cross bar 82, the resilient force of hook 30 will not drop the angled front portion of cross bar 72 into notch 50.

Thus, the latching mechanism of the present invention has been cycled from the position of Figure 8 when aneurysm clip 26 is loaded into jaws 14 and 20; to the position of Figure 3 as the surgical assistant partially closes handles 16 and 22 until cross bar 72 engages notch 50. As the surgeon further closes handles 16 and 22 toward one another, the position of cross bars 72 and 82 is as shown in Figures 5 and 6. As the surgeon relaxes the grip on handles 16 and 22, cross bar 72 recycles over cross bar 82 into the original position shown in Figures 7 and 8.

If, after setting clip 26, the surgeon wishes to reposition or even withdraw it, he can easily do so without removing forceps 10 from the incision. Open jaws 14 and 20 can be placed about clip 26 and closed until cross bar 72 engages notch 50 indicating to the surgeon that clip 26 is secured in jaws 14 and 20. The surgeon then closes handles 16 and 20 to open jaws 27, 28 of clip 26 for repositioning. If the surgeon wishes to completely remove clip 26, one may follow the above steps for repositioning and continue by withdrawing jaws 27, 28 away from the anatomy about which they were positioned and then relaxing the grasp on handles 16, 22 to close jaws 27, 28 and thus recycle hook 30 over latch 40 behind notch 50 and then partially closing handles 16, 22 to engage cross bar 72 in notch 50. The surgeon, knowing that clip 26 is firmly held in jaws 14, 20 and that jaws 27, 28 are closed, can withdraw clip 26 with jaws 27, 28 closed so as to avoid damage to the surrounding anatomy during withdrawal.

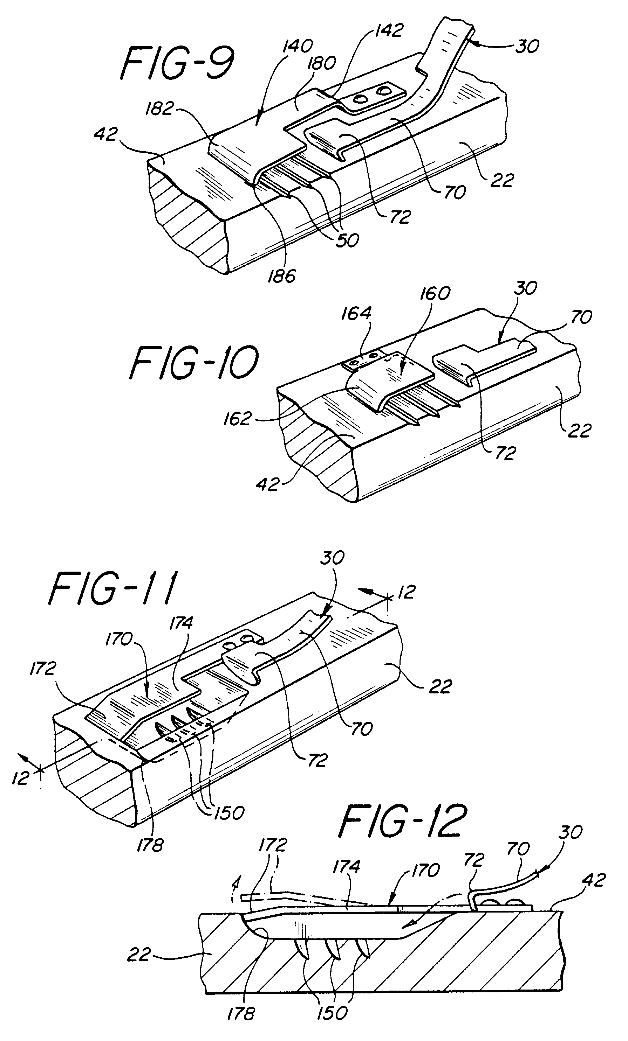

Referring now to Figure 9 there is shown an alternative embodiment of the invention using a different latch 140 with a hook 30 which is the same as the hook 30 in the embodiment of Figure 3. Latch 140 is rigidly affixed to the inside surface 42 of handle 22 just proximally of notches 50. Shaft 180 and cross bar 182 of latch 140 extend across notches 50 and the free end 186 of latch 140 is biased into contact with surface 42 of handle 22. There is a rising portion 142 of latch 140 distally of the attachment point of latch 140 to permit shaft 180 and cross bar 182 to be spaced above surface 42 to permit cross bar 72 of hook 30 to ride under latch 140 to engage notches 50. A plurality of notches may be used to provide multiple retaining positions for the latch mechanism and, thus, multiple positions for jaws 14 and 20 of forceps 10.

Alternatively as shown in Figure 11, notches 50 need not be grooves extending all the way across handle 22 but may be one or more half moon shaped notches 150 recessed into a portion of surface 42.

Alternatively, latch 140 may be replaced by latch 160 as shown in Figure 10 with a cross bar 162 and an attachment tab 164 extending from the side of latch 160. This embodiment can use half moon shaped notches 150 which do not extend all the way across surface 42 (see Figure 11).

In another embodiment shown in Figures 11 and 12 latch 170 may be attached to surface 42 extending over a recess 172 in surface 42 and having its distal end aligned with surface 42 for smooth action of the mechanism. Latch 170 is substantially straight and does not have the rising bend as like bend 142 of latch 140. Cross bar 174 extends part way across recess 172 but not so far that it interferes with shaft portion 70 of hook 30. One or more half moon shaped notches, 150 are placed in the bottom of recess 172 to retain cross bar 72 of hook 30. As cross bar 72 moves distally as handles 16 and 22 close toward one another, cross bar 72 slides up a ramp 178 at the distal end of recess 172 passing under cross bar 174. As handles 16 and 22 open again, cross bar 72 passes over the top of latch 170 without engaging notches 150 or cross bar 174 to recycle the latch mechanism in the same fashion as the latch mechanism of Figure 3 can be recycled.

In an embodiment where either latches 140, 160 or 170 are used, proximal extensions 60 and 62 shown in Figure 1 could be replaced by a compression or torsion spring 23 mounted between members 12 and 18 near pivot point 24. Alternatively, extensions 60 and 62 could be turned around to face distally and be contained within the space defined by handles 16 and 22. Also if extensions 60 and 62 are omitted, cross bar 72 can be adjusted to hook onto the proximal surface 43 of distal portion 41 of latch 40 (see Fig. 3). This could be used to provide the first predetermined spacing of jaws 14, 20 to allow clip 26 to be placed inside the jaws 14, 20.

The present invention has been described in conjunction with the preferred embodiments. Those skilled in the art will appreciate that many modifications and changes may be made to the preferred embodiments without departing from the present invention. It is, therefore, not intended to limit the present invention except to set forth in the appended claims.

## Claims

1. In a forceps-type surgical instrument (10) having first and second members (12, 18) each having a jaw (14, 20) disposed in opposing relationship on a distal portion thereof and each having a handle (16, 22) disposed in opposing relationship on a proximal portion thereof said members (12, 18) pivotably connected together so that when said handles are closed toward one another said jaws (14, 20) close toward one another,
including
a recyclable latching mechanism (40; 140; 160; 170) having at least a first position for disposing said jaws (14, 20) at a first predetermined spacing and at least a second position for disposing said jaws (14, 20) at a second predetermined spacing comprising:
retaining means (50, 150) on said handle (22) of said second member (18);
a latch means (40; 140; 160; 170) attached to the handle (22) of said second member (18) and being resiliently flexible in the plane of motion of said handles (16, 22) and having a distal end and a proximal end, a distal portion (82; 162; 172; 182) of said latch means (40; 140; 160; 170) extending at least partially about said retaining means;
a hook means (30) attached to the handle (16) of said first member (12) and resiliently flexible in the plane of motion of the handles (16, 22) and having a distal end and a proximal end;
means for biasing said handles (16, 22) in a first predetermined position;
said hook means (30) and said retaining means (50, 150) cooperating to dispose said handles (16, 22) in a second predetermined position upon further closing of the handles (16, 22);
said hook means (30) and said latch means (40, 140, 160, 180) cooperating upon further closing and then opening of said handles (16, 22) to return said jaws (14, 20) to said first position without said hook means engaging said retaining means (50, 150),
characterised in that
said retaining means on said handle (22) of said second member (18) includes at least one V-shaped notch (50, 150);
said hook means (30) includes an L-shaped projection (70, 72) extending longitudinally from the distal end thereof, said hook means L-shaped projection having a cross bar portion (72) extending transversely of the handle (22) of said second member (18);
said latch means (40) includes an L-shaped projection (80, 82) extending longitudinally from the distal end thereof, said latch means L-shaped projection having a cross bar portion (82) extending transversely of the handle (22) of said second member (18);
the cross bar portions (72, 82) of said latch means projection and said hook means projection extend toward one another and are disposed in partially interfering relationship so that when said distal end of said hook means moves distally, only the two cross bar portions (72, 82) of said latch means projection and said hook means projection will contact one another and only during a portion of the moved distance said hook means cross bar (72) moves through said notch (50).

2. The instrument of claim 1 wherein a proximal portion (52) of said notch (50) forms an acute angle with the adjoining inside (42) of said handle (22) of said second member (18) and the distal portion (54) of said notch (50) forms an obtuse angle with the adjoining inside (42) of said handle (22) of said second member (18).

3. The instrument of claim 1 wherein said latch means projection cross bar (82, 162, 172, 182) is spaced distally from said notch (50, 150).

4. The instrument of claim 1 wherein said cross bar portion (82) of said latch means (40; 140) has a distal end and a proximal end, the distal end of said latch means cross bar (82) biased in contact with said second member handle (16) and the proximal portion of said latch means cross bar spaced longitudinally from the distal end of said latch means; and,
connecting means (80; 180) connecting said latch means cross bar means (82; 182) to the remainder of said latch means (40; 140) so that the proximal portion of said latch means cross bar means is spaced longitudinally from the distal end of said latch means.

5. The instrument of claim 4 wherein the surface of said latch means cross bar (82) facing the opposing handle (22) of said second member (18) has a convex curve (84) as one proceeds from the distal to the proximal portion of said latch means cross bar (82).

6. The instrument of claim 1 wherein said cross bar portion (72) of said hook means (30) has a distal end and a proximal end, the distal end of said hook means cross bar (72) biased in contact with said second member handle (22) and the proximal portion of said hook means cross bar (72) spaced longitudinally from the distal end of said hook means; and,
connecting means (70) connecting said hook means cross bar (72) to the remainder of said hook means so that the proximal portion of said hook means cross bar is spaced longitudinally from the distal end of said hook means.

7. The instrument of claim 6 wherein the distal portion of said hook means cross bar (72) includes a member (86) projecting toward said second handle (22) and forming an acute angle with the remainder (70) of said hook means cross bar (72).

8. The instrument of claim 1 wherein said biasing means includes a further portion (62) of said latch means (40) extending proximally beyond the proximal end of said second handle (22); and,
a further portion (60) of said hook means (30) extending proximally beyond the proximal end of said first handle (16);
the proximal ends (64, 66) of said further portions (60, 62) of said latch means (40) and said hook means (30) releasably connected together.

9. The instrument of claim 8 wherein the means for releasably connecting said further portions (60, 62) of said latch means (40) and said hook means (30) releasably together includes a generally rectangular opening (64) in the proximal portion (62) of one of said further portions and a tab (66) having an expanded head (68) on the proximal portion (60) of said other further portion.

10. The instrument of claim 1 further including means for providing an audible signal indicating the movement of said latching mechanism (40; 140; 160; 170) into said predetermined positions.

11. The instrument of claim 1 further including means for providing a tactile signal indicating the movement of said latching mechanism into said predetermined positions.

12. The instrument of claim 1 wherein said means for biasing said handles in a first position is integral with at least a part of said latching mechanism.

13. The instrument of claim 1 wherein said latch means (40) includes a leaf spring having a distal end and a proximal end and whose proximal end is attached at a proximal end of said second member handle (22) and whose distal end extends beyond said retaining means (50; 150).

14. The instrument of claim 1 wherein said hook means (30) includes a leaf spring having a proximal end and a distal end whose proximal end is attached to the proximal end of said first member handle (16) and whose distal end extends toward the opposing inside surfaces (42) of said second handle (22) and whose distal end is positioned to engage at least a portion of said latch means (40, 140, 160, 170, 180) when said handles (16, 22) close from said first predetermined position.

15. The instrument of claim 1 further including a longitudinally extending recess (172);
wherein said retaining means (150) are placed in the base of said recess (172);
wherein said latch means (170) includes a piece of flat spring steel attached to said second member (18) proximally of said recess (172) and includes a distal portion extending over said recess (172);
whereby said hook means (30) enters said recess (172) and engages said retaining means (150) as said handles (16, 22) are closed together; and
said hook means (30) exit said recess (172) in the distal direction as said handles (16, 22) are closed further together.

16. The instrument of claim 1 wherein said latch means (140; 160) includes a short leaf spring material attached to the handle (22) of the second member (18) just proximally of said retaining means (50);
a portion (142) of said latch means (140) raised above the confronting surface (42) of said second handle member (18); and,
the distal end (162; 182) of said latch means (140; 160) resiliently engaging the opposing inside surface (42) of said second handle member (18) at a point placed distally of said retaining means (50).

17. The instrument of claim 1 wherein said latch means (160) includes a piece of leaf spring material attached to the handle (22) of said second member (18) beside said retaining means (50);
said latch means (160) has a tab (164) extending from one side thereof and a cross bar portion (162) integrally attached to said tab (164) and raised above the confronting surface (42) of said second member (18) and said cross bar (162) having a distal end in resilient contact with said second member (18) at a point disposed distally of said retaining means (50).

18. The instrument of claim 1 wherein said latch means (170) includes a short piece of leaf spring material attached to said second handle member (18) just proximally of said retaining means (150); and
wherein said retaining means (150) includes a recess (172) disposed below the surface (42) of said second handle member (18) and includes at least one engaging notch (50) in the base thereof.

## Patentansprüche

1. Zangenartiges chirurgisches Instrument (10) mit einem ersten und einem zweiten Teil (12, 18), die jeweils eine Backe (14, 20) aufweisen, die an einem distalen Bereich davon einander gegenüberliegend angeordnet sind, und jeweils einen Handgriff (16, 22) aufweisen, die an einem proximalen Bereich davon einander gegenüberliegend angeordnet sind, wobei die Teile (12, 18) schwenkbar miteinander verbunden sind, so daß, wenn die Handgriffe einander angenähert werden, sich die Backen (14, 20) gegeneinander schließen, mit
einem rückführbaren Verriegelungsmechanismus (40, 140; 160; 170) mit zumindest einer ersten Stellung zur Verlagerung der Backen (14, 20) zu einem ersten vorbestimmten Abstand und zumindest einer zweiten Stellung zur Verlagerung der Backen (14, 20) zu einem zweiten vorbestimmten Abstand, aufweisend:
eine Rückhalteeinrichtung (50, 150) auf dem Handgriff (22) des zweiten Teils (18);
eine Rastklinkeneinrichtung (40; 140; 160; 170), die an dem Handgriff (22) des zweiten Teils (18) befestigt ist und elastisch in der Bewegungsebene der Handgriffe (16, 22) auslenkbar ist mit einem distalen Ende und einem proximalen Ende, wobei ein distaler Bereich (82; 162; 172; 182) der Rastklinkeneinrichtung (40; 140; 160; 170) sich zumindest teilweise um die Rückhalteeinrichtung herum erstreckt;
eine Hakeneinrichtung (30), die am Handgriff (16) des ersten Teils (12) befestigt ist und elastisch in der Bewegungsebene der Handgriffe (16, 22) auslenkbar ist, mit einem distalen Ende und einem proximalen Ende;
eine Einrichtung zum Spannen der Handgriffe (16, 22) in einer ersten vorbestimmten Position;
wobei die Hakeneinrichtung (30) und die Rückhalteeinrichtung (50, 150) zusammenwirken, um die Handgriffe (16, 22) bei weiterer Annäherung der Handgriffe (16, 22) in eine zweite vorbestimmte Position zu verlagern;
wobei die Hakeneinrichtung (30) und die Rastklinkeneinrichtung (40, 140, 160, 180) bei weiterer Schließung und anschließendem Öffnen der Handgriffe (16, 22) zusammenwirken, um die Backen (14, 20) in die erste Position zurückzubewegen, ohne daß die Hakeneinrichtung in die Rückhalteeinrichtung (50, 150) eingreift,
**dadurch gekennzeichnet, daß**
die Rückhalteeinrichtung auf dem Handgriff (22) des zweiten Teils (18) zumindest eine V-förmige Kerbe (50, 150) aufweist;
die Hakeneinrichtung (30) einen L-förmigen Vorsprung (70, 72) aufweist, der sich von deren distalem Ende in Längsrichtung erstreckt, wobei der L-förmige Vorsprung der Hakeneinrichtung einen Riegelbereich (72) aufweist, der sich quer zum Handgriff (22) des zweiten Teils (18) erstreckt;
die Rastklinkeneinrichtung (40) einen L-förmigen Vorsprung (80, 82) aufweist, der sich von deren distalem Ende in Längsrichtung erstreckt, wobei der L-förmige Vorsprung der Rastklinkeneinrichtung einen Riegelbereich (82) aufweist, der sich quer zum Handgriff (22) des zweiten Teils (18) erstreckt;
die Riegelbereiche (72, 82) des Vorsprungs der Rastklinkeneinrichtung und des Vorsprungs der Hakeneinrichtung sich zueinander erstrecken und zumindest teilweise überlappend angeordnet sind, so daß, wenn das distale Ende der Hakeneinrichtung sich distal bewegt, nur die beiden Riegelbereiche (72, 82) des Vorsprungs der Rastklinkeneinrichtung und des Vorsprungs der Hakeneinrichtung miteinander in Kontakt kommen und der Riegel (72) der Hakeneinrichtung sich nur während eines Teils der bei der Bewegung zurückgelegten Distanz durch die Kerbe (50) bewegt.

2. Instrument nach Anspruch 1, bei dem ein proximaler Bereich (52) der Kerbe (50) mit der angrenzenden Innenseite (42) des Handgriffs (22) des zweiten Teils (18) einen spitzen Winkel und der distale Bereich (54) der Kerbe (50) mit der angrenzenden Innenseite (42) des Handgriffs (22) des zweiten Teils (18) einen stumpfen Winkel bildet.

3. Instrument nach Anspruch 1, bei dem der Riegelvorsprung (82, 162, 172, 182) der Rastklinkeneinrichtung distal von der Kerbe (50, 150) beabstandet ist.

4. Instrument nach Anspruch 1, bei dem der Riegelbereich (82) der Rastklinkeneinrichtung (40; 140) ein distales Ende und ein proximales Ende aufweist, wobei das distale Ende des Riegels (82) der Rastklinkeneinrichtung in Kontakt mit dem Handgriff (16) des zweiten Teils vorgespannt ist, und der proximale Teil des Riegels der Rastklinkeneinrichtung in Längsrichtung vom distalen Ende der Rastklinkeneinrichtung beabstandet ist; und
eine Verbindungseinrichtung (80; 180), die den Riegel (82; 182) der Rastklinkeneinrichtung mit dem Rest der Rastklinkeneinrichtung verbindet, so daß der proximale Bereich des Riegels der Rastklinkeneinrichtung in Längsrichtung vom distalen Ende der Rastklinkeneinrichtung beabstandet ist.

5. Instrument nach Anspruch 4, bei dem die Oberfläche des Riegels (82) der Rastklinkeneinrichtung (40, 140), die dem gegenüberliegenden Handgriff (22) des zweiten Teils (18) zugewandt ist, eine konvexe Krümmung (84) aufweist, wenn man vom distalen zum proximalen Bereich des Riegels (82) der Rastklinkeneinrichtung vorschreitet.

6. Instrument nach Anspruch 1, bei dem der Riegelbereich (72) der Hakeneinrichtung (30) ein distales Ende und ein proximales Ende aufweist, wobei das distale Ende des Riegels (72) der Hakeneinrichtung in Kontakt mit dem Handgriff (22) des zweiten Teils vorgespannt ist und der proximale Bereich des Riegels (72) der Hakeneinrichtung in Längsrichtung vom distalen Ende der Hakeneinrichtung beabstandet ist; und
eine Verbindungseinrichtung (70) aufweist, die den Riegel (72) der Hakeneinrichtung mit dem Rest der Hakeneinrichtung verbindet, so daß der proximale Bereich des Riegels der Hakeneinrich tung in Längsrichtung vom distalen Ende der Hakeneinrichtung beabstandet ist.

7. Instrument nach Anspruch 6, bei dem der distale Bereich des Riegels (72) der Hakeneinrichtung einen zum zweiten Handgriff (22) vorspringenden Teil (86) aufweist und einen spitzen Winkel mit dem Rest (70) des Riegels (72) der Hakeneinrichtung bildet.

8. Instrument nach Anspruch 1, bei dem die Spanneinrichtung einen weiteren Teil (62) der Rastklinkeneinrichtung (40) aufweist, der sich proximal über das proximale Ende des zweiten Handgriffs (22) hinaus erstreckt; und
einen weiteren Bereich (60) der Hakeneneinrichtung (30), der sich proximal über das proximale Ende des ersten Handgriffs (16) hinaus erstreckt; wobei die proximalen Enden (64, 66) der weiteren Bereiche (60, 62) der Rastklinkeneinrichtung (40) und der Hakeneinrichtung (30) lösbar miteinander verbunden sind.

9. Instrument nach Anspruch 8, bei dem die Einrichtung zur lösbaren Verbindung der weiteren Bereiche (60, 62) der Rastklinkeneinrichtung (40) und der Hakeneinrichtung (30) eine im wesentlichen rechteckförmige Öffnung (64) im proximalen Bereich (62) von einem der weiteren Bereiche und eine Zunge (66) aufweist mit einem erweiterten Kopf (68) auf dem proximalen Bereich (60) des anderen weiteren Bereichs.

10. Instrument nach Anspruch 1, weiterhin aufweisend eine Einrichtung zur Schaffung eines hörbaren Signals, das die Bewegung des Verriegelungsmechanismus (40, 140; 160; 170) in die vorbestimmten Positionen anzeigt.

11. Instrument nach Anspruch 1, weiterhin aufweisend eine Einrichtung zur Schaffung eines spürbaren Signals, das die Bewegung des Verriegelungsmechanismus in die vorbestimmten Positionen anzeigt.

12. Instrument nach Anspruch 1, bei dem die Einrichtung zum Spannen der Handgriffe in eine erste Position mit zumindest einem Teil des Verriegelungsmechanismus einteilig ausgebildet ist.

13. Instrument nach Anspruch 1, bei dem die Rastklinkeneinrichtung (40) eine Blattfeder mit einem distalen Ende und einem proximalen Ende aufweist, deren proximales Ende an einem proximalen Ende des Handgriffs (22) des zweiten Teils befestigt ist, und deren distales Ende sich über die Rückhalteeinrichtung (50; 150) hinaus erstreckt.

14. Instrument nach Anspruch 1, bei dem die Hakeneinrichtung (30) eine Blattfeder aufweist mit einem proximalen Ende und einem distalen Ende, deren proximales Ende am proximalen Ende des Handgriffs (16) des ersten Teils befestigt ist, und deren distales Ende sich zur gegenüberliegenden Innenseitenfläche (42) des zweiten Handgriffs (22) erstreckt, und deren distales Ende positioniert ist, um zumindest einen Bereich der Rastklinkeneinrichtung (40, 140, 160, 170, 180) zu erfassen, wenn die Handgriffe (16, 22) aus ihrer ersten vorbestimmten Position sich einander annähern.

15. Instrument nach Anspruch 1, weiterhin aufweisend einen sich in Längsrichtung erstreckenden Rezeß (172);
bei dem die Rückhalteeinrichtung (150) in der Basis des Rezeß (172) angeordnet ist;
bei dem die Rastklinkeneinrichtung (170) ein Stück flachen Federstahls aufweist, das am zweiten Teil (18) proximal zum Rezeß (172) befestigt ist, und einen distalen Bereich aufweist, der sich über den Rezeß (172) erstreckt;
wobei die Hakeneinrichtung (30) in den Rezeß (172) eintritt und die Rückhalteeinrichtung (150) erfaßt, wenn die Handgriffe (16, 22) einander angenähert werden; und
die Hakeneinrichtung (30) den Rezeß (172) in distaler Richtung verläßt, wenn die Handgriffe (16, 22) weiter aneinander angenähert werden.

16. Instrument nach Anspruch 1, bei dem die Rastklinkeneinrichtung (140; 160) ein kurzes Blattfedermaterial aufweist, das am Handgriff (22) des zweiten Teils (18) gerade proximal zur Rückhalteeinrichtung (50) befestigt ist;
ein Bereich (142) der Rastklinkeneinrichtung (140) über die Gegenfläche (42) des Handgriffs des zweiten Teils (18) erhaben ist; und
das distale Ende (162, 182) der Rastklinkeneinrichtung (140; 160) elastisch die gegenüberliegende Innenfläche (42) des Handgriffs des zweiten Teils (18) an einer Stelle erfaßt, die distal zur Rückhalteeinrichtung (50) angeordnet ist.

17. Instrument nach Anspruch 1, bei dem die Rastklinkeneinrichtung (160) ein Stück eines Blattfedermaterials aufweist, das am Handgriff (22) des zweiten Teils (18) neben der Rückhalteeinrichtung (50) befestigt ist;
die Rastklinkeneinrichtung (160) eine Zunge (164) aufweist, die sich zu einer Seite davon weg erstreckt und einen Riegelbereich (162), der integral an der Zunge (164) befestigt ist und über die Gegenfläche (42) des zweiten Teils (18) erhaben ist, wobei der Riegel (162) mit einem distalen Ende in elastischem Kontakt mit dem zweiten Teil (18) an einer Stelle ist, die distal zur Rückhalteeinrichtung (50) gelegen ist.

18. Instrument nach Anspruch 1, bei dem die Rastklinkeneinrichtung (170) ein kurzes Stück eines Blattfedermaterials aufweist, das am Handgriff des zweiten Teils (18) gerade proximal zur Rückhalteeinrichtung (150) befestigt ist; und bei dem die Rückhalteeinrichtung (150) einen Rezeß (172) aufweist, der unterhalb der Oberfläche (42) des Handgriffs des zweiten Teils (18) angeordnet ist und zumindest eine Eingriffskerbe (50) in dessen Basis aufweist.

## Revendications

1. Dans un instrument chirurgical (10) de type forceps, comportant des premier et deuxième organes (12, 18) ayant chacun une mâchoire (14, 20), disposée en relation opposée sur une partie distale de ceux-ci et ayant chacun une poignée (16, 22), disposée en relation opposée sur une partie proximale de ceux-ci, lesdits organes (12, 18) étant reliés ensemble a' pivotement de manière que, lorsque lesdites poignées sent fermées l'une en direction de l'autre, lesdites mâchoires (14, 20) se ferment, en direction l'un de l'autre,
comprenant,
un mécanisme de verrouillage (40; 140; 160; 170) à rappel, ayant au moins une première position pour disposer lesdites mâchoires 14, 20 en un premier espacement prédéterminé et au moins une deuxième position pour disposer lesdites mâchoires (14, 20) en un deuxième espacement prédéterminé, comprenant :
un moyen de retenue (50, 150) réalisé sur ladite poignée (22) dudit second organe (18);
un moyen de verrouillage (40; 140; 160; 170) fixé a la poignée (22) dudit second organe (18) et flexible élastiquement dans le plan de déplacement desdites poignées (16, 22) et ayant une extrémité distale et une extrémité proximale, une partie distale (88; 162; 172; 182) lesdits moyens de verrouillage (40; 140; 160; 170) s'tendant au moins partiellement autour dudit moyen de retenue. Un moyen formant crochet (30) fixé à la poignée, (16) dudit premier organe (12) est flexible élastiquement dans le plan de déplacement des poignées (16, 22) et ayant une extrémité distale et une extrémité proximale;
des moyens pour déplacer lesdites poignées (16, 22) dans une première position prédéterminée;
lesdits moyens formant crochet (30) et lesdits moyens de retenue (50, 150) coopérant pour disposer lesdites poignées (16,22) en une deuxième position prédéterminée lors d'une fermeture ultérieure des poignées (16, 22); ledit moyen formant crochet (30) et ledit moyen de verrouillage (40; 140; 160; 180) coopérant, lors d'une ouverture plus marquée et d'une ouverture subséquente desdites poignées (16, 22), afin de faire revenir lesdites mâchoires (14, 20) à ladite première position, sans que lesdits moyens formant crochet viennent s'engager sur lesdits moyens de retenue (50, 150),
caractérisé en ce que ledit moyen de retenue réalise sur ladite poignée (22) dudit second organe (18), comprend au moins une encoche en V (50; 150);
ledit moyen formant crochet (30) comprenant une saillie en L (76, 72), s'étendant longitudinalement, depuis son extrémité distale, ladite saillie en L du moyen formant crochet ayant une partie de barrette transversale (72) s'étendant dans la direction transversale de la poignee (22) dudit second organe (18);
ledit moyen de verrouillage (40) comprenant une saillie en L (80, 82) s'étendant en direction longitudinale depuis son extrémité distale, ladite saillie en L de moyen de verrouillage ayant une partie de barrette transversale (82) s'étendant dans la direction transversale de la poignée (22) dudit second organe (18);
les parties de barrette transversale (72, 82) de ladite saillie de moyen de verrouillage et de ladite saillie de moyen formant crochet s'étendant en direction l'une de l'autre et étant disposées en une relation les faisant interférer partiellement de manière que lorsque ladite extrémité distale desdits moyen formant crochet se déplace distalement, seules les deux parties de barrette transversale (72, 82) de ladite saillie de moyen de verrouillage et de ladite saillie de moyen formant crochet entre en contact l'une de l'autre et que, seulement sur une partie de la distance, ladite barrette transversale (72) du moyen formant crochet se déplace a travers ladite encoche (50).

2. L'instrument de la revendication 1, dans lequel la partie proximale (52) de ladite encoche (50) forme un angle aigu avec l'intérieur (42) connexe de ladite poignée (22) dudit second organe (18) et la partie distale (54) de ladite encoche (50) forme un angle obtus avec l'intérieur (42) connexe de ladite poignée (22) dudit second organe (18).

3. L'instrument de la revendication 1 dans lequel ladite barrette transversale (82, 162, 172, 182) formant saillie sur le moyen de verrouillage est espacée distalement de ladite encoche (50, 150).

4. L'instrument de la revendication (1) dans lequel ladite partie de barrette transversale (82) dudit moyen de verrouillage (40; 140) présente une extrémité distale et une extrémité proximale, l'extrémité distale de ladite barrette transversale (82) du moyen de verrouillage étant déplacée pour venir en contact avec ladite poignée (16) du second organe et la partie proximale de ladite barrette transversale de moyen de verrouillage étant espacée longitudinalement par rapport à l'extrémité distale dudit moyen de verrouillage; et
des moyens de liaison (80; 180) reliant lesdits moyens à barrette transversale (82; 182) des moyens de verrouillage au reste desdits moyens le verouillage (40; 140) de manière que la partie proximale desdits moyens à barrette transversale des moyens de verrouillage soit espacée longitudinalement de l'extrémité distale dudit moyen de verrouillage.

5. L'instrument de la revendication 1, dans lequel la surface de la barrette transversale (82) des moyens de verrouillage tournée vers la poignée (22) opposée dudit second organe 18 présente une incurvation (84) convexe, lorsque l'on passage de la partie distale à la partie proximale de ladite barrette transversale (82) de moyen de verrouillage.

6. L'instrument de la revendication 1, dans lequel ladite partie de barrette transversale (72) dudit moyen formant crochet (30) comporte une extrémité distale et une extrémité proximale, l'extrémité distale de ladite barrette transversale (72) de moyen formant crochet étant déformée pour venir en contact avec ladite poignée (22) de second organe et la partie proximale de ladite barrette transversale (72) de moyen formant crochant étant espacée longitudinalement de l'extrémité distale dudit moyen de forment crochet; et
un moyen de liaison (70) relie ladite barrette transversale (72) de moyen formant crochet au reste de ladite barrette transversale de moyen formant crochet étant espacée longitudinalement de l'extrémité distale dudit moyen formant crochet.

7. L'instrument de la revendication 6, dans lequel la partie distale de ladite barrette transversale (72) de moyen formant crochet comprend un élément (86) faisant saillie en direction de ladite seconde poignée (22) et formant un angle aigu avec le restant (70) de ladite barrette transversale (72) de moyen formant crochet.

8. L'instrument de la revendication 1, dans lequel ledit moyen de déplacement comprend une partie supplémentaire (62) dudit moyen de verrouillage (40), s'étendant de façon proximale au-delà de l'extrémité proximale de ladite seconde poignée (22); et
une partie supplémentaire (60) dudit moyen formant crochet (30) s'étendant de facon proximale au-delà de l'extrémité proximale de ladite première poignée (16);
les extrémités proximales (64, 66) desdites parties supplémentaires (60, 62) desdits moyens de verrouillage (40) et desdits moyens formant crochet (30) étant relié ensemble de façon désolidarisable.

9. L'instrument de la revendication 8, dans lequel le moyen assurant la liaison désolidarisable entre lesdites parties supplémentaires (60, 62) dudit moyen de verrouillage (40) et dudit moyen formant crochet (30) comprend une ouverture (64) de forme généralement rectangulaire, ménagée dans la partie proximale (62) de l'une desdites parties supplémentaires, et une patte (66) ayant un tête (68) déployée et réalisée sur la partie proximale (60) de ladite autre partie supplémentaire.

10. L'instrument de la revendication 1, comprenant en outre un moyen pour produire un signal audible, indiquant la déplacement dudit mécanisme de verrouillage (40; 140; 160; 170) dans les dites positions prédéterminées.

11. L'instrument de la revendication 1, comprenant en outre un moyen pour produire un signal tactile indiquant le déplacement dudit mécanisme de verrouillage dans lesdites position prédéterminées.

12. L'instrument de la revendication 1, dans lequel ledit moyen destiné à déplacer lesdites poignées dans une première position est réalisé d'un seul tenant avec au moins une partie dudit mécanisme de verrouillage.

13. L'instrument de la revendication 1, dans lequel ledit moyen de verrouillage (40) comprend un ressort à lame ayant une extrémité distale et une extrémité proximale et dont l'extrémité proximale est fixée à une extrémité proximale de ladite poignée (22) de second organe et dont l'extrémité distale s'étend au-delà dudit moyen de retenue (50; 150).

14. L'instrument de la revendication 1, dans lequel ledit moyen formant crochet (30) comprend un ressort à lame ayant un extrémité proximale et une extrémité distale dont l'extrémité proximale est fixée à l'extrémité proximale de ladite poignée (16) de premier organe et dont l'extrémité distale s'étend en direction des surfaces intérieures (42) opposées de ladite seconde poignée (22) et dont l'extrémité distale est positionnée de façon à venir au contact d'au moins une partie desdits moyens de verrouillage (40; 140; 160; 170; 180), lorsque lesdites poignées (16, 22) se ferment, à partir de ladite première position prédéterminée.

15. L'instrument de la revendication 1, comprenant en outre une cavité (162) s'étendant longitudinalement;
dans lequel ledit moyen de retenue (150) est placé dans la base de ladite cavité (172);
dans lequel ledit moyen de verrouillage (170) comprend un morceau en acier à ressort plat fixé audit second organe (18) à proximité de ladite cavité (72) et comprenant une partie distale s'étendant sur ladite cavité (172);
de manière que ledit moyen formant crochet (30) pénètre dans ladite cavité (172) et vienne au contact dudit moyen de retenue (150), lorsque lesdites poignées (16, 22) sont refermées l'une sur l'autre; et,
ledit moyen formant crochet (30) sortant de ladite cavité (172) dans la direction distale lorsque lesdites poignées (16, 22) sont fermées, l'une sur l'autre, subséquemment.

16. L'instrument de la revendication 1, dans lequel ledit moyen de verrouillage (140; 160) comprend un matériau court formant ressort à lame, fixé à la poignée (22) du second organe (18) juste a proximité dudit moyen de retenue (50);
une partie (142) dudit moyen de verrouillage (140) montant sur la surface (42) places en regard dudit second organe formant poignée (18); et l'extrémité distale (162, 182) dudit moyen de verrouillage (140; 160) venant élastiquement en contact sur la surface intérieure (42) placée en regard dudit second organe formant poignée (18) en un point situé distalement dudit moyen de retenue (50).

17. L'instrument de la revendication 1, dans lequel ledit moyen de verrouillage (160) comprend un morceau de matériau faisant ressort à lame, fixé à la poignée (22) dudit second organe (1), à côté dudit moyen de retenue (50);
ledit moyen de retenue (160) comporte une patte (140) s'étendant depuis l'un de ces côtés et une partie de barrette transversale (162) reliée d'un seul tenant à ladite patte (164) et montant au-dessus de la surface (42) placée en regard dudit second organe (18) et ladite barrette transversale (162) ayant une extrémité distale placée en contact élastique avec ledit second organe (18) en un point situé distalement par rapport audit moyen de retenue (50).

18. L'instrument de la revendication 1, dans lequel ledit moyen de verrouillage (170) comprend un morceau court d'un matériau formant ressort à lame fixé audit organe formant seconde poignée (18), juste à proximité dudit moyen de retenue (150); et
dans lequel ledit moyen de retenue (150) comprend une cavité (172) disposée au-dessous de la surface (42) dudit second organe formant poignée (18) et comprend au moins une encoche (50) d'engagement, dans sa base.
